(19) **Europäisches Patentamt / European Patent Office / Office européen des brevets**

(11) **EP 2 151 194 A1**

(12) # EUROPEAN PATENT APPLICATION
published in accordance with Art. 153(4) EPC

(43) Date of publication:
**10.02.2010 Bulletin 2010/06**

(51) Int Cl.:
**A61B 8/06** (2006.01)

(21) Application number: **08710927.8**

(22) Date of filing: **07.02.2008**

(86) International application number:
**PCT/JP2008/052048**

(87) International publication number:
**WO 2008/136201 (13.11.2008 Gazette 2008/46)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **27.04.2007 JP 2007117954**

(71) Applicant: **Hitachi Medical Corporation
Chiyoda-ku
Tokyo 101-0021 (JP)**

(72) Inventors:
• **YOSHIKAWA, Hideki
Tokyo 100-8220 (JP)**
• **AZUMA, Takashi
Tokyo 100-8220 (JP)**

(74) Representative: **MERH-IP
Matias Erny Reichl Hoffmann
Paul-Heyse-Strasse 29
80336 München (DE)**

(54) **ULTRASONIC DIAGNOSTIC APPARATUS**

(57)    An ultrasonic diagnostic apparatus, including a first imaging unit for configuring plural pieces of first-image data based on reception signals received by an ultrasound probe, a velocity vector detector for measuring blood and tissue velocity vectors based on the plural pieces of first-image data inputted from the first imaging unit, a blood-flow image extracting unit for configuring a blood-flow image based on the velocity vectors measured, a histogram unit for calculating number of pixels for each brightness with respect to the blood-flow image configured, a threshold controller for inputting a brightness threshold value, an information processing unit for color-mapping pixels whose brightness are higher than the brightness threshold value, and configuring second-image data by adding the pixels to at least the one piece of first-image data, and a display for displaying the second-image data configured.

FIG.7

**Description**

CROSS-REFERENCES TO RELATED APPLICATIONS

This application relates to and claims priority from Japanese Patent Application No. 2007-117954, filed on April 27, 2007, the entire disclosure of which is incorporated herein by reference.

TECHNICAL FIELD

The present invention relates to an apparatus for taking advantage of an ultrasound signal transmitted/received by an ultrasound probe, and measuring the spatial distribution of brightness of a blood-flow image and velocity vector of the blood flow, and identifying, imaging, and displaying a stagnation region in which the blood flow stagnates.

BACKGROUND ART

Myocardial infarction and cerebral infarction, symptoms of which are caused to occur by interruption of a blood flow, are serious diseases whose mortality rates are high next to cancer, and whose symptoms are caused to occur in an unexpected and sudden manner. Here, the interruption of a blood flow is attributed to a plaque on a blood-vessel wall, or a blood clot due to exfoliation of the plaque or stagnation of the blood flow. Meanwhile, the plaque itself is formed in such a manner that the plaque grows gradually on the blood-vessel wall portion with a lapse of a few months to a few years. On account of this situation, it is important to identify and monitor, at the stage of medical check, a location at which the plaque is in a danger of being likely to be formed. Simultaneously, it is requested to take a preventive measure against the formation of the plaque before it results in the occurrence of a clogging of the blood vessel.

The size and growth rate of a plaque under observation are conceivable as judgment criterions as to whether or not to carry out a medical treatment against the plaque. What becomes an important indicator in trying to diagnose a plaque is the blood-flow state on the periphery of the plaque; in particular, the velocity distribution of the blood flow.

The technology for measuring the velocity distribution of a blood flow based on autocorrelation operation, and implementing the two-dimensional imaging of the velocity distribution is a technology widely installed into ultrasonic diagnostic apparatuses (e.g., Japanese Patent No. 3370780).

Patent Document 1 Japanese Patent No. 3370780

DISCLOSURE OF THE INVENTION

PROBLEM TO BE SOLVED BY THE INVENTION

In the above-described conventional technology, the problem of visualizing the stagnation of a blood flow still remains unsolved. Accordingly, an object of the present invention is to provide an ultrasonic diagnostic apparatus for displaying a blood-flow distribution image which indicates the stagnation region.

MEANS FOR SOLVING THE PROBLEM

As an embodiment, an ultrasonic diagnostic apparatus of the present invention includes an ultrasound probe for transmitting/receiving an ultrasound to/from an object, a transmitting beamformer for allocating a predetermined time delay to each of piezoelectric elements in order to form a desired transmission beam, each of the piezoelectric elements constituting the ultrasound probe, a D/A converter for converting a digital signal from the transmitting beamformer to an analog signal, a TGC (: Time Gain Controller) for correcting an amplitude attenuation which occurs in the propagation process, an A/D converter for converting a reception signal to a digital signal, a receiving beamformer for aligning the phase of the reception signal obtained in each element of the ultrasound probe, and correcting a time difference which occurs due to each element position, an envelope detector for detecting a RF signal outputted from the receiving beamformer, and converting the RF signal to an image signal, a scan converter for configuring plural pieces of two-dimensional image data by using the image signal transmitted from the envelope detector, a frame memory for storing at least the two pieces of image data, a velocity vector detector for measuring blood-flow and tissue velocity vectors which have occurred between the plural pieces of image data stored, a blood-flow image extracting unit for configuring a blood-flow image, the blood-flow image being acquired by extracting a blood-flow region out of the image data on the basis of the velocity vectors measured, a frequency-distribution calculation unit for calculating frequency distribution of brightness of the blood-flow image on the blood-flow image, a displayed-brightness control unit for allowing an operator to set a brightness threshold value for pixels of brightness which are to be color-mapped on a brightness's frequency-distribution table displayed on a screen, a blood flow imaging unit for configuring a blood-flow distribution image by color-coding the pixels of the brightness in accordance with the brightness on the image data, the brightness having exceeded the brightness threshold value set in the displayed-brightness control unit by the operator, and a display for displaying the blood-flow distribution image.

Here, the measurement of the velocity vectors may also be made for each region which is acquired by segmentalizing the image data. Also, the blood-flow region may be extracted out of the image data on the basis of the measurement result of the velocity vectors. Also, the frequency distribution of the brightness of the blood-flow image may be measured. Also, a desired brightness may be selected by the operator as the threshold value for the pixels of the brightness which are to be color-mapped on the blood-flow distribution image on the brightness's frequency-distribution table displayed on the screen. Moreover, the pixels may be assumed to have brightness which have exceeded the desired brightness. Also, information displayed in a manner of being superimposed on the image data is assumed to be either or both of the velocity vector of the blood flow and the brightness distribution of the blood flow. Furthermore, the information displayed at one time may be freely selected by the operator. Also, there may be provided a blood-flow distribution image display, including a unit for measuring the velocity vectors from the plural pieces of image data, a unit for extracting the blood-flow region out of the image data by using the velocity vectors measured, and a unit for calculating the frequency distribution of the brightness of the blood-flow region, and color-mapping the pixels whose brightness have exceeded the threshold value for the brightness set by the operator, wherein the blood-flow distribution image display displays the blood-flow distribution image. Here, the information selected from the image data, the velocity vector of the blood flow, and the brightness distribution of the blood flow by the operator, such as the brightness distribution and the blood-flow vector indicating blood-flow concentration and blood-flow velocity, are displayed on the blood-flow distribution image in a manner of being superimposed on the image data.

As another embodiment, an ultrasonic diagnostic apparatus of the present invention includes an ultrasound probe for transmitting/receiving ultrasound signals to/from an object, a first imaging unit for configuring plural pieces of first-image data based on the reception signals received by the ultrasound probe, a velocity vector detector for measuring blood and tissue velocity vectors based on the plural pieces of first-image data inputted from the first imaging unit, a blood-flow image extracting unit for configuring a blood-flow image based on the velocity vectors measured by the velocity vector detector, a histogram unit for calculating number of pixels for each brightness with respect to the blood-flow image configured by the blood-flow image extracting unit, a threshold controller for inputting a brightness threshold value, an information processing unit for color-mapping pixels whose brightness are higher than the brightness threshold value, and configuring second-image data by adding the pixels to at least the one piece of first-image data, and a display for displaying the second-image data configured by the information processing unit.

As still another embodiment, an ultrasonic diagnostic apparatus of the present invention includes an ultrasound probe for transmitting/receiving ultrasound signals to/from an object, a first imaging unit for configuring plural pieces of first-image data based on the reception signals received by the ultrasound probe, a memory for memorizing at least two pieces of the plural pieces of first-image data, each of the plural pieces of first-image data corresponding to each of the plural reception signals ranging from the 1st signal to the n-th signal and received by the ultrasound probe, a velocity vector detector for measuring blood and tissue velocity vectors from the first-image data corresponding to the (n-1)-th signal and the first-image data corresponding to the n-th signal, these two pieces of first-image data being inputted from the first imaging unit and being memorized into the memory, an image accumulation unit for creating an accumulation image by adding the plural pieces of first-image data to each other, a blood-flow image extracting unit for extracting a blood-flow image based on the velocity vectors measured by the velocity vector detector, or the accumulation image created by the image accumulation unit, a histogram unit for calculating number of pixels for each brightness with respect to the accumulation image, a threshold controller for inputting a brightness threshold value, an information processing unit for color-mapping pixels whose brightness are higher than the brightness threshold value, and configuring second-image data by adding the pixels to at least the one piece of first-image data, and a display for displaying the second-image data configured by the information processing unit.

ADVANTAGES OF THE INVENTION

According to the ultrasonic diagnostic apparatus of the present invention, it becomes possible to implement color mapping of the blood-flow brightness distribution for indicating blood-flow concentration and blood-flow flow velocity, and to display the blood-flow distribution image where a stagnation region is emphasized.
The other objects, features, and advantages of the present invention will become apparent from the following description of embodiments of the present invention associated with the accompanying drawings.

BEST MODE FOR CARRYING OUT THE INVENTION

In the ultrasonic diagnostic apparatus of the present invention, the velocity vectors of an object are measured using plural pieces of image data. Next, based on the measurement result, a blood-flow region is extracted out of the image data. Moreover, a frequency-distribution table is calculated which indicates the frequency distribution of brightness of the blood-flow region. Finally, based on the frequency distribution of the brightness, pixels whose brightness have exceeded a brightness threshold value set by the operator are color-mapped on the image data, thereby implementing and displaying the blood-flow distribution image.

EMBODIMENT 1

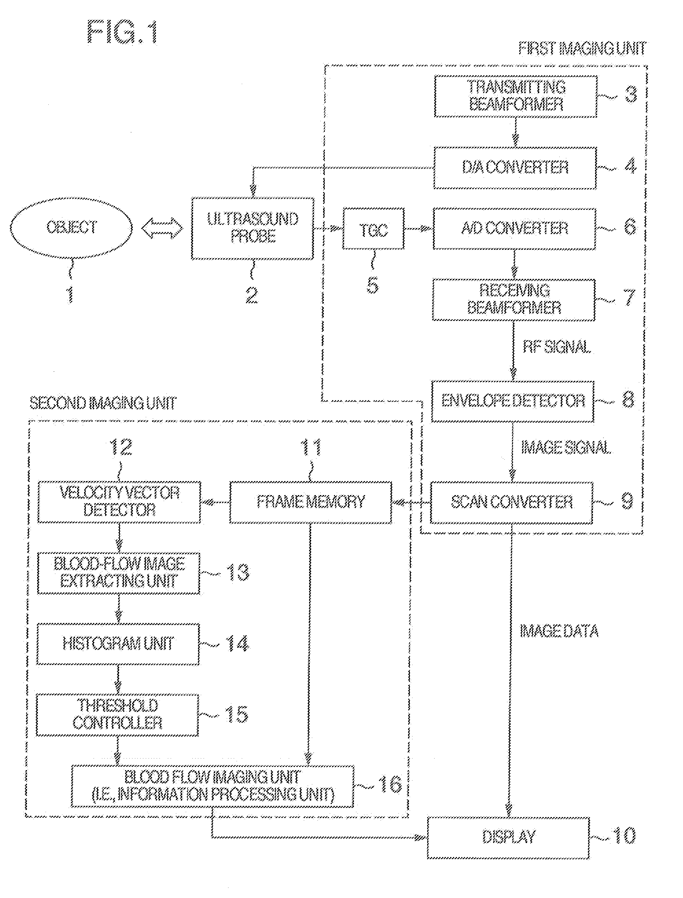

Fig. 1 is a block diagram for illustrating the configuration of an ultrasonic diagnostic apparatus according to a first embodiment of the present invention. First, the explanation will be given below concerning processing steps in a first imaging unit for configuring a B-mode image which becomes source data for measuring the blood-flow distribution. Subsequently, the explanation will be given below regarding processing steps in a second imaging unit for configuring the blood-flow distribution image using the B-mode image inputted from the first imaging unit.

The processing steps in the first imaging unit for configuring the B-mode image are the well-known technology. Accordingly, the explanation thereof will be given here briefly.

The ultrasound irradiation surface of an ultrasound probe 2 is configured such that a plurality of piezoelectric elements are arranged in a line. Each piezoelectric element is in charge of transmitting/receiving the ultrasound. A voltage pulse from a transmitting beamformer 3 is inputted into each piezoelectric element via a D/A converter 4. The resultant piezoelectric vibration of each piezoelectric element generates the ultrasound, then irradiating the ultrasound towards an object 1. At this time, a predetermined time delay is electronically allocated to each piezoelectric element. The ultrasound transmitted from each piezoelectric element is focused at a predetermined position inside the object 1. A reflection echo reflected from the object 1 is received by each piezoelectric element. In order to correct an attenuation amount in the reflection-echo signal which occurs in the propagation process, a TGC (: Time Gain Controller) 5 makes an amplitude correction thereto in correspondence with the propagation distance. Subsequently, the reception signal is transmitted to a receiving beamformer 7 via an A/D converter 6. The receiving beamformer 7 multiples the reception signal by a delay time in correspondence with the distance from the focus position to each piezoelectric element, and adds the resultant delay-time-multiplied reception signals to each other, then outputting this addition result (i.e., phase-aligned addition). This transmission/reception of the ultrasound is performed in all of scanning lines along the line of the piezoelectric elements. This operation allows acquisition of the two-dimensional reflection echo distribution of the object 1. A RF signal separated into a real part and an imaginary part is outputted from the receiving beamformer 7, then being transmitted to an envelope detector 8. The RF signal transmitted to the envelope detector 8, after being converted into an image signal, is subjected to an inter-scanning-lines pixel interpolation by a scan converter 9. In this way, the image signal, after being reconfigured into two-dimensional image data, is displayed on a display 10.

Subsequently, the explanation will be given below regarding the processing steps in the second imaging unit for configuring the blood-flow distribution image.

At least two pieces of image data outputted from the scan converter 9 are stored into a frame memory 11. The two pieces of image data are transmitted to a velocity vector detector 12. The velocity vector detector 12 measures blood-flow and tissue velocity vectors which have occurred during the acquisitions of the two pieces of image data. Based on the blood-flow velocity vector measured, a blood-flow image extracting unit 13 configures a blood-flow image in which only a blood-flow region is extracted out of the image data. The velocity of the blood flow is larger as compared with the velocity of the tissue by an amount of one digit or more. As a result, the blood-flow region can be extracted by allocating a certain constant threshold value to the velocity vector measured, and selecting an region having a velocity vector larger than the constant threshold value. Subsequently, the blood-flow image is transmitted to a histogram unit 14, which calculates the frequency distribution of brightness of the blood-flow image. Based on the frequency distribution of the brightness measured, a threshold controller 15 allows the operator to input a brightness which becomes the threshold value for pixels which are to be color-mapped. Here, the image data stored into the frame memory 11 are transmitted not only to the velocity vector detector 12, but also to a blood flow imaging unit (i.e., information processing unit) 16. The blood flow imaging unit 16 selects, from the blood-flow image, the pixels whose brightness have exceeded the threshold value inputted in the threshold controller 15 by the operator. After that, the unit 16 color-codes these pixels in accordance with the brightness thereof. Finally, the unit 16 superimposes the resultant color-coded blood-flow image on the image data read from the frame memory 11, thereby allowing the configuration of the blood-flow distribution image. The blood-flow distribution image is transmitted to the display 10, then being displayed on the display.

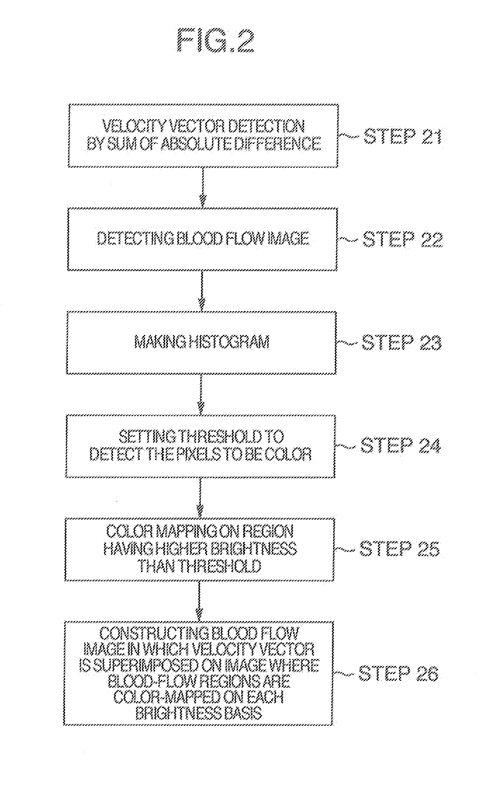

Next, in accordance with a processing-step table illustrated in Fig. 2, the explanation will be given below concerning the processing steps up to the processing step at which the blood-flow distribution image in which the brightness distribution of the blood flow is color-mapped is configured from the image data fetched from the scan converter 9 in the blood flow imaging unit 16.

It is assumed that the two pieces of image data stored into the frame memory 11 are $f_1$ and $f_2$ in the order in which they are stored therein. Namely, it is assumed that the image data $f_2$ is the newest image data fetched. At a step 21, the measurement of the velocity vector is performed using these two pieces of image data. When the two pieces of image data are inputted into the velocity vector detector 12, the image data $f_2$ is segmentalized into regions (which, hereinafter, will be referred to as "segmentalized regions") for measuring the velocity vector. It is desirable that these segmentalized regions be the smallest possible regions implementable in order to determine how finely the distribution of the velocity vector is measured. These segmentalized regions, however, are required to be larger than such molecules as hemoglobin and lipid, i.e., reflection-echo sources flowing in the blood flow. This is because such molecules are searched and traced in the measurement of the velocity vector. The size of each segmentalized region is determined by frequency of the ultrasound irradiated from the ultrasound probe, and diameter width and focus position of the ultrasound probe. Accordingly, under a condition of about 7-MHz frequency, about 3-cm diameter width, and about 2-cm-depth focus position, the size of each segmentalized region becomes equal to 500 μm in square.

Consequently, the operator can freely modify the size of each segmentalized region under the condition that 500μm in square is employed as its initial value.

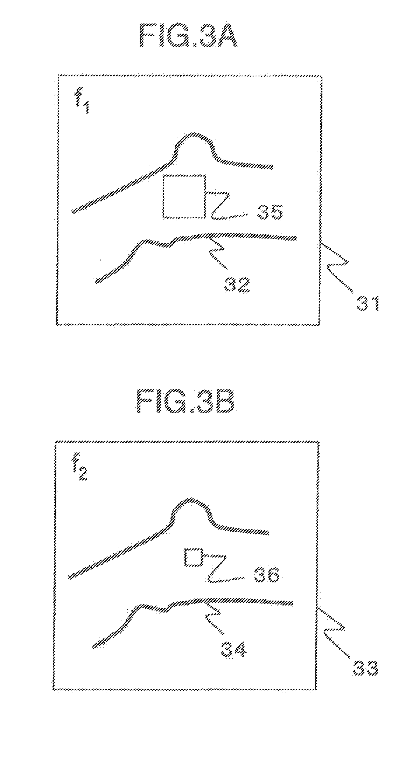

Also, it is assumed that the operator can arbitrarily set the initial value as well. Next, referring to Fig. 3A and Fig. 3B, the explanation will be given below regarding the measurement methodology for the velocity vector, focusing attention on a single segmentalized region subf$_2$ set on the image data $f_2$. In the drawings, the reference numerals 31 and 33 denote the image data $f_1$ and $f_2$, respectively, and 32 and 34 denote blood vessels on the image data $f_1$ and $f_2$, respectively. The segmentalized region subf$_2$ (36) is set on the image data $f_2$ (33). Moreover, a searching region 35 for searching for an region (i.e., matching region) which matches the segmentalized region subf$_2$ (36) most satisfactorily is set at a position on the image data $f_1$ at which the central point of the searching region 35 becomes the same as the central point of the segmentalized region subf$_2$ (36). The size of the searching region 35 is determined by the velocity of the blood flow and a frame rate at which the image data are to be acquired. Enlarging the searching region 35, however, results in an increase in the processing time by the amount equivalent thereto. Accordingly, the searching region 35 is required to be set into the smallest possible region implementable. In the case of, e.g., a lower-limb vein, the blood-flow velocity is equal to about 1 to 2 cm/second, which is exceedingly slow.

Consequently, when fetching the 300-frame-per-second image data, a movement of the blood flow which occurs between the image data becomes equal to about 300 to 600μm. Accordingly, a searching region equivalent to this movement will be set.

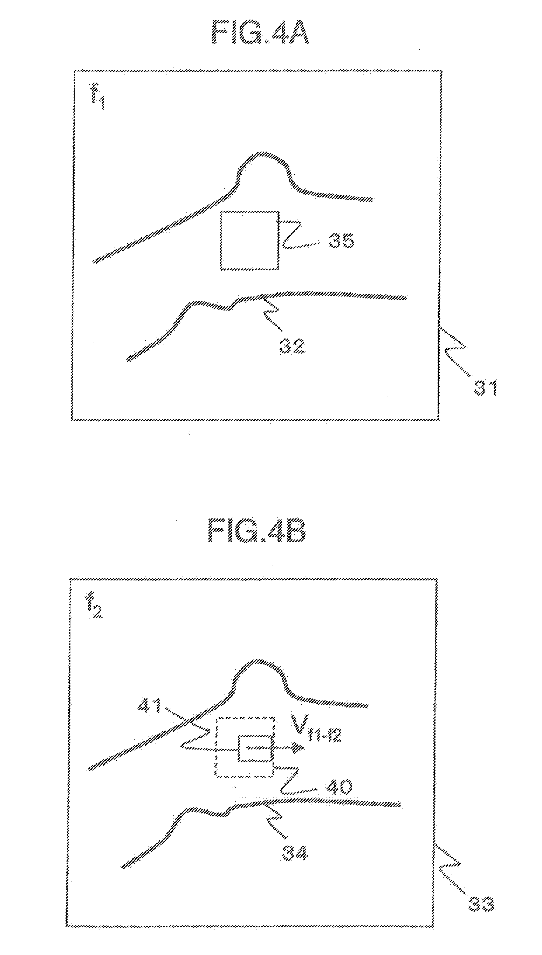

In the case of setting a searching region, the size of the searching region can be optimized by estimating a velocity vector, which is to be measured based on the next image data, from a velocity vector which has been measured based on the previous image data. For example, as illustrated in Fig. 4A and Fig. 4B, it is assumed that a velocity vector $V_{f1-f2}$ has been measured from the image data $f_1$ and $f_2$. Next, if image data $f_3$ is newly acquired, a segmentalized region is set on the image data $f_3$, and a searching region is set on the image data $f_2$. Moreover, a velocity vector which has occurred between the image data $f_2$ and $f_3$ is measured based on basically the same methodology. At this time, if the frame rate is sufficiently faster as compared with the velocity of the movement of the object, it can be estimated that the measurement result of this velocity vector will turn out to be a measurement result which is close to $V_{f1-f2}$.

Accordingly, the searching region which is to be set on the image data $f_2$ from the essential viewpoint is set such that the central point of the searching region is the same as the central point of the searching region 35 set on the image data $f_1$, and such that the size of the searching region is the same as the size of the searching region 35 (i.e., before-optimization searching region 40). From the above-described estimation, however, it is possible to set a searching region whose size is maintained in the direction of $V_{f1-f2}$, and whose size is reduced in another direction into, e.g., one-half of the region which should be set from the essential viewpoint (i.e., after-optimization searching region 41). The optimization of the searching region narrows the range in which a region which matches the segmentalized region is to be searched for. This condition makes it possible to shorten a processing time needed for the measurement of the velocity vector.

The position of the matching region is defined as follows: While shifting the segmentalized region subf$_2$ (36) within the searching region 35 on a one-pixel-by-one-pixel basis, the total sum $c_{x,y}$ of differential absolute values of brightness in respective positions (x, y) within the searching region 35 is determined in accordance with the following Expression (1): Then, the position of the matching region is defined as being a position at which this total sum $c_{x,y}$ becomes equal to its minimum.

[Expression 1]

$$c_{x,y} = \sum_{l}^{N_l} \sum_{m}^{N_m} |[subf_2 (l, m, t) - subf_2 (l, m, t-\delta t)]|$$

(1)

Here, ($N_l$, $N_m$) and (l, m) denote the pixel count of the segmentalized region subf$_2$ (36) and the relative pixel position in each position (x, y) within the searching region 35, respectively. The distance ranging from the central point of the segmentalized region to the central point of the matching region is the movement of the object in the segmentalized region which has occurred between the two pieces of image data. The velocity vector can be determined from the value of this distance. The explanation has been given here regarding the typical example of methodologies for searching for the matching region. The operation methodologies, however, are not limited thereto, as long as they are methods for searching for a region which matches a certain specific region most satisfactorily. Examples of such other operation methodologies are least-squares method and correlation method. The search for the matching region explained so far is performed with respect to all of the segmentalized regions set on the image data $f_2$. This processing allows implementation of the measurement of the velocity vector for the entire image data.

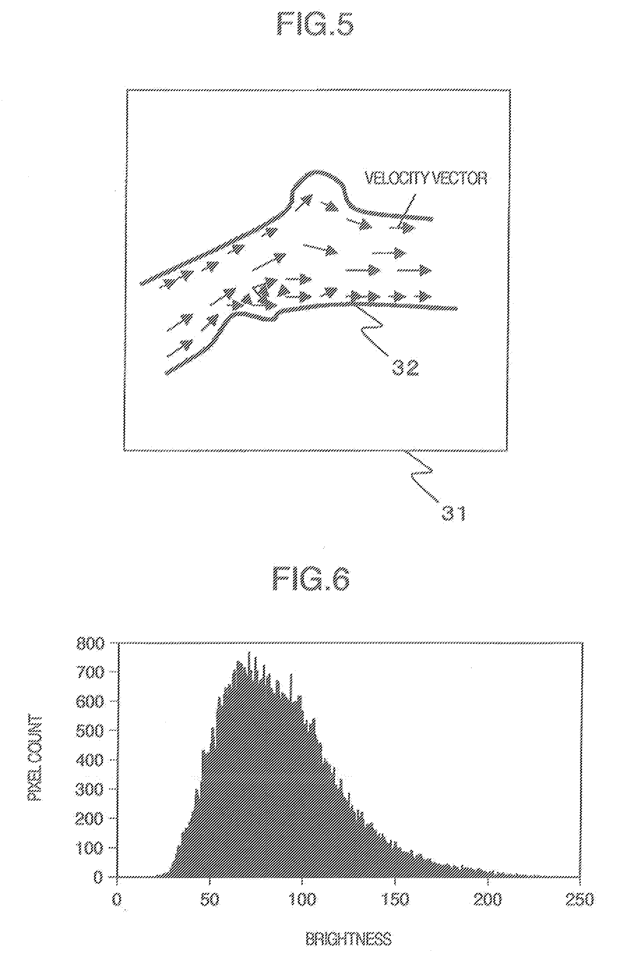

Fig. 5 illustrates a typical diagram for indicating the measurement result of the velocity vector. The blood vessel 32 exists on the image data 31, which becomes the criterion in trying to measure the velocity vector. The velocity vector is indicated by an arrow in the drawing. The size of the arrow indicates the magnitude of the blood-flow velocity. Usually, the blood-flow velocity on the blood-vessel wall side becomes smaller as compared with the blood-flow velocity at the blood-vessel central portion. Also, the tissue is at rest under an ideal condition that the ultrasound probe is fixed. As a result, the measurement result of the velocity vector becomes equal to substantially zero in the regions other than the blood flow.

At a step 22, the blood-flow region is extracted based on the velocity vector measured. When the movement of the tissue is sufficiently smaller as compared with the movement of the blood flow, an region in which the velocity vector is equal to substantially zero is eliminated from the image data $f_2$. This processing allows the configuration of the blood-flow image in which only the blood-flow region is extracted. For example, in a neck artery and a lower-limb vein, i.e., important targets in the plaque diagnosis, the velocity of the blood flow is larger as compared with the velocity of the tissue by an amount of about one digit. Consequently, the extraction of the blood-flow region based on the velocity vector is comparatively easy.

As the extraction method for the blood-flow region, the explanation has been given here concerning the methodology of utilizing the measurement result of the velocity vector obtained using the two pieces of image data. The image data to be utilized for the measurement of the velocity vector, however, are not limited to the two pieces. Namely, velocity vectors between the respective image data may be measured using still more pieces of image data. Then, from the plurality of velocity vectors measured in the segmentalized regions set up at the same position of the respective image data, an average velocity vector at this same position is calculated. Next, the blood-flow region may be judged from the magnitude of the average velocity vector. In this case, the blood-flow region can be distinguished without being influenced by the instantaneous movement of the tissue. Also, the time-series velocity vectors measured among the plural pieces of image data are accumulation-added to each other in each segmentalized region. This processing makes it possible to clarify more accurately the difference in the velocity between the blood-flow region and the tissue region.

Also, the irradiation with a low-frequency component ultrasound is included into the wave-transmission sequence. Then, the tissue region can also be judged from image data which is acquired by the low-frequency-component ultrasound irradiation. In the low-frequency-component ultrasound irradiation, the reception signal of a sufficiently strong intensity cannot be acquired from the reflection-echo sources contained in the blood flow. As a result, the resultant configured image data turns out to be an image where the tissue component is emphasized. Accordingly, selecting the low-brightness region on the image makes it possible to extract the blood-flow region. In the case of this wave-transmission sequence, the measurement of the velocity vector is not necessarily required. In this case, it turns out that the velocity vector of the blood flow cannot be displayed, and that only the brightness-distribution image of the blood flow is displayed. By the amount equivalent thereto, however, a tremendous shortening in the processing time can be expected.

Also, the use of image data configured with the high-frequency component allows the blood-flow region to be imaged with the brightness which is higher as compared with that of the tissue region by an amount of one digit or more. As a result, extracting the high-brightness region on the image makes it possible to create the blood-flow region. The use of a contrast agent is also effective in amplifying the reflection-echo intensity from the blood-flow region. In this case as well, the measurement of the velocity vector is not necessarily required, and the shortening in the processing time can be expected. Also, subtracting the image configured with the low-frequency component from the image configured with the high-frequency component is more effective in extracting the blood-flow region.

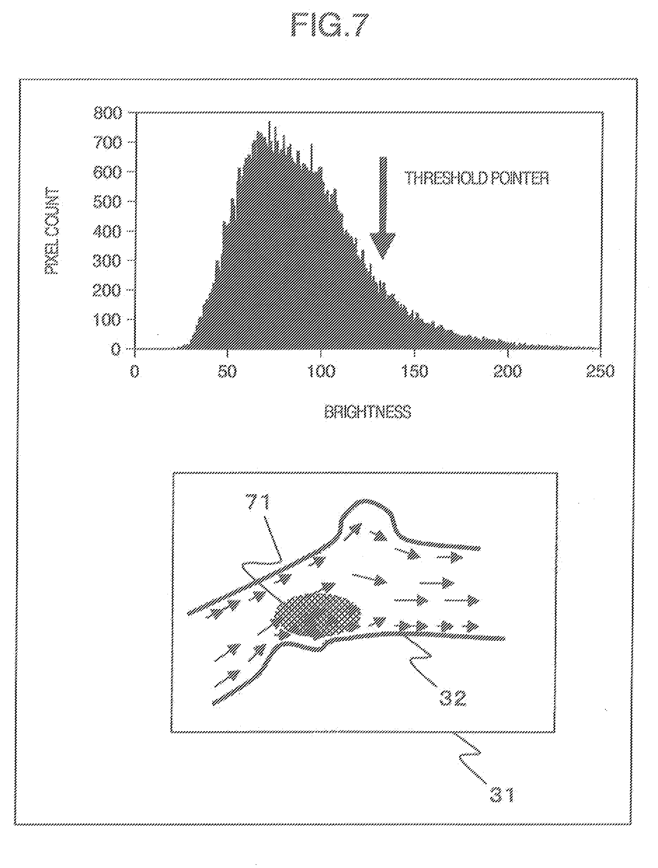

Next, the frequency distribution of the brightness of the blood-flow image is calculated (step 23). The frequency distribution table displays the brightness (which is equal to 0 to 255 at the maximum in the case of 8-bit image data) in the horizontal axis, and the accumulation of the pixels having each brightness in the vertical axis (Fig. 6). Based on the frequency distribution table, the operator inputs a brightness threshold value which becomes the criterion for determining pixels which are to be color-mapped (step 24). The brightness on the screen of the table is the intensity of the reflection-echo signal accumulated on each pixel in a unit time. Namely, the brightness indicates a location into which inflow quantity of the blood flow is large, or a location at which the blood flow stagnates. Accordingly, adjusting the threshold value allows implementation of, e.g., an image display where only the location at which the blood flow stagnates is emphasized. An example of the input method is as follows: The frequency distribution table and a threshold pointer for setting the threshold value are displayed on the display 10 in accompaniment with the image. Then, the reference line is moved onto a desired position, thereby setting the threshold value (Fig. 7). On the screen, in accompaniment with the movement of the reference line operated by the operator, the color mapping of the blood-flow distribution image is also switched by being caused to instantaneously respond thereto. Also, it is possible to select a specific region on the image data, e.g., the blood-vessel central region, and to select the brightness of the specific region as the threshold value. Basically, the blood-flow velocity at the blood-vessel central portion is higher as compared with the blood-flow velocity on the blood-vessel wall side. Consequently, the blood-vessel central portion exhibits the low brightness on the image data. As a result, by selecting the brightness at the blood-vessel center or in proximity to the center as the reference (i.e., threshold value), it becomes possible to implement the color mapping of the brightness distribution of the entire blood-flow region.

At a step 26, the pixels whose brightness have exceeded the threshold value selected at a step 25 are color-coded on the image data $f_2$. Moreover, depending on the request by the operator, it is possible to display the color-coded pixels in such a manner that the velocity vector measured by the velocity vector detector 12 is superimposed on the color-coded pixels. This superimposition display makes it possible to expect, e.g., clarification of the cause or mechanism for the stagnation of the blood flow in the entire blood flow within the blood vessel. Also, this superimposition display allows implementation of imaging of blood-flow meander due to blood-vessel tumor, and flow-in of the blood flow into aneurysm. This successful implementation of the imaging makes it possible to expect an enhancement in the diagnosis capability for the blood-vessel abnormalities and the like.

Fig. 7 illustrates an example of the blood-flow distribution image displayed on the display 10. The frequency distribution table and the velocity vector are displayed on the original image data $f_2$ (33). In addition thereto, a portion (denoted by 71 in the drawing) is color-mapped where the blood flow stagnates and which has become a high-brightness region. Incidentally, the operator can freely modify the measurement region, thereby being capable of shortening the processing time. Also, the mode of the image data is not particularly limited (like, e.g., the image configured with the fundamental-frequency component and the image configured with the high-frequency component), as long as the reflection-echo intensity from the blood flow is sufficiently strong. Also, the use of a contrast agent amplifies the reflection-echo intensity, which makes it possible to display the blood-flow distribution image with an even higher accuracy.

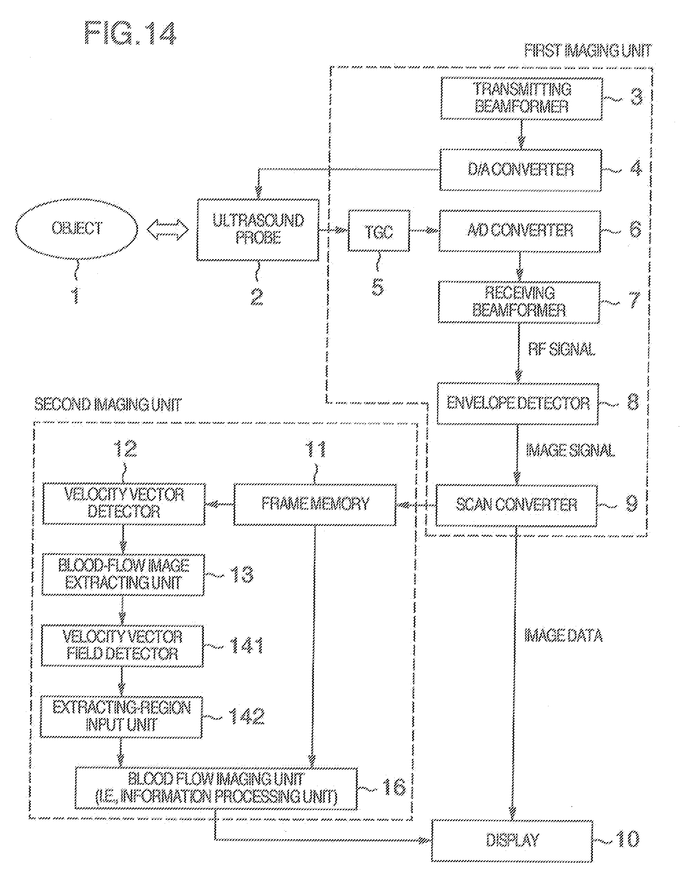

In the above-described processing, the velocity vector of the blood flow is used in order to distinguish the blood-flow region. Meanwhile, the blood-flow state, which is the direct element to be imaged, is judged using the brightness distribution of the blood flow on the image data. Here, a processing which is more effective than the use of the brightness distribution is made executable by judging the turbulent flow of the blood flow from the velocity vector thereof. Fig. 14 illustrates a block diagram of a device configuration example in this case. In Fig. 14, of the block diagram of the device configuration example illustrated in Fig. 1, the histogram unit 14 is replaced by a velocity vector field detector 141.

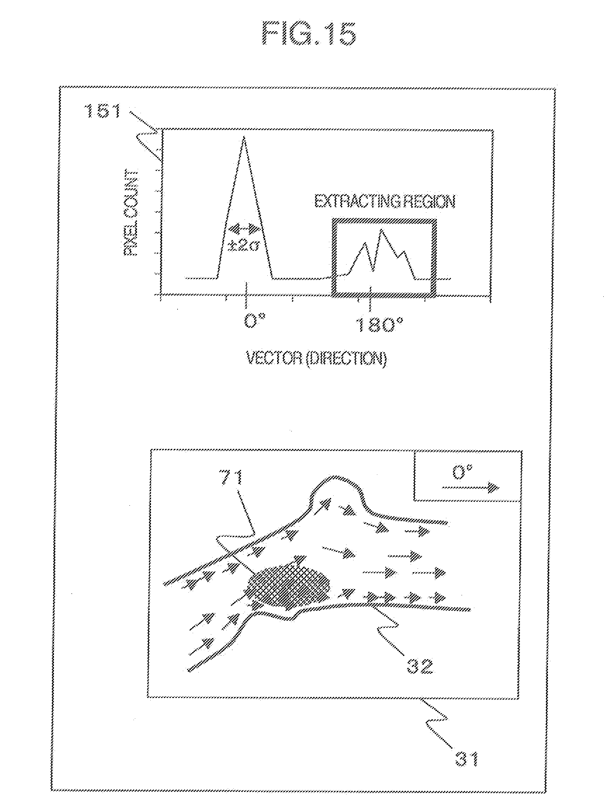

As illustrated in Fig. 15, the velocity vector field detector 141 configures directions of measured vectors in the horizontal axis, and, in the vertical axis, vector distribution 151 where frequencies of the directions are assumed. The directions of the vectors are set based on the image data 31 displayed on the display in accompaniment therewith. Namely, for example, the right direction is selected as the reference value and defined as being 0 °. The directions of the blood flows are basically oriented into one and the same direction. Accordingly, the vector distribution exhibits a high frequency in this one and the same direction. In a turbulent-flow region, however, the directions of the vectors are oriented into various directions. Consequently, the vector distribution exhibits a frequency in a direction other than 0°. An extracting region 152 is provided in the vector distribution 151. The operator adjusts this extraction region 152 properly in an Extracting-region input unit 142, thereby making it possible to select a vector having a desired direction or pixels corresponding thereto. The selected pixels on the image data are color-coded by the blood flow imaging unit 16, then being displayed on the display.

Also, variance value ($\sigma$) of each peak is measured from the vector distribution 151. Then, an region where the variance value is large can be selected as an region where the directions of the vectors are nonuniform, i.e., a turbulent-flow region. When a tumor exists on a blood vessel locally, and when a turbulent flow on the periphery is wished to be extracted, many of the vectors possess a uniform direction. Accordingly, the variance value of a peak in this uniform direction is small, and exhibits a large frequency. Consequently, the turbulent flow can be extracted by extracting an region other than a range (e.g., $\pm 2\sigma$ in Fig. 15) which is determined by the variance value of this peak.

Also, the mutual degree of similarity of pixels is determined using adjacent blood-flow vectors and the mutual correlation operation. Then, pixels are selected whose degree of similarity is found to be lower than a threshold value set in advance. This processing also makes it possible to distinguish the pixels in the turbulent-flow region. In this case, the adjustment of the threshold value or the extracting region is not necessarily required.

EMBODIMENT 2

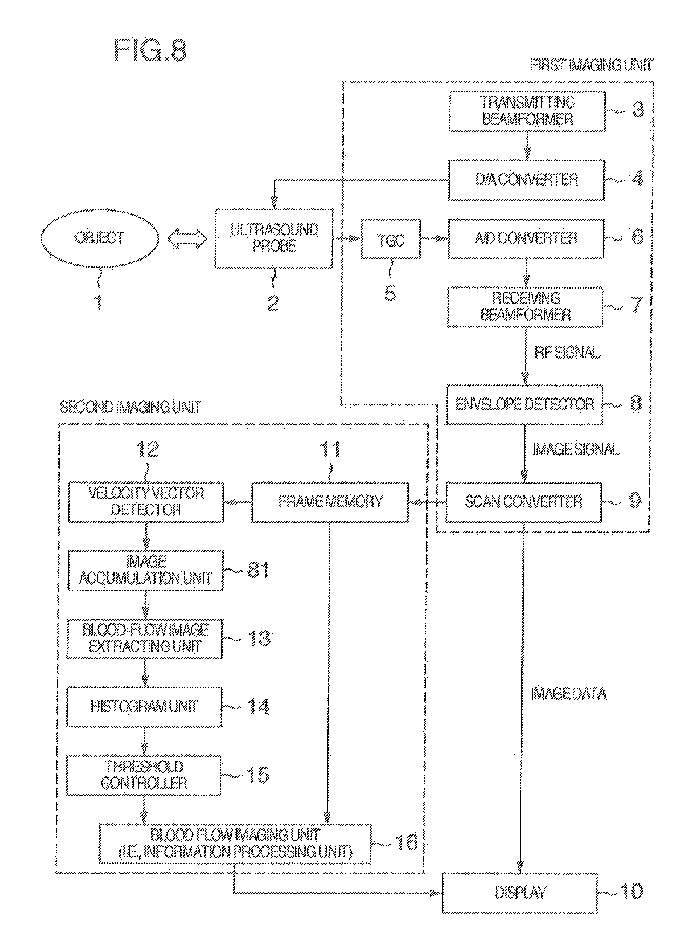

Fig. 8 is a block diagram for illustrating the configuration of an ultrasonic diagnostic apparatus according to a second embodiment of the present invention. The device configuration and processing steps in the first imaging unit are the same as the ones in the first embodiment. Accordingly, the explanation will be given below from the second imaging unit.

As is the case with the first embodiment, at least the two pieces of image data are stored into the frame memory 11. In the second embodiment, however, n (n > 1) pieces of image data ($f_l$ - $f_n$), which are set by the operator, are stored into the frame memory 11 one after another. Here, it is assumed that the image data $f_n$ is the newest of the n pieces of image data fetched into the frame memory 11. The velocity vector detector 12 measures velocity vectors which have occurred among plural pieces of image data, or desirably, among all of the n pieces of image data fetched into the frame memory 11. The measurement methodology for the velocity vectors is the same as the measurement methodology in the first embodiment.

In the ultrasonic diagnostic apparatus according to the second embodiment, an accumulation image is configured by applying an addition processing to plural pieces of image data on the basis of the velocity vectors measured. Then, the accumulation image configured is utilized for the measurement of the brightness distribution. Also, the accumulation image is utilized for the extraction of the blood-flow region in the blood-flow image extracting unit 13.

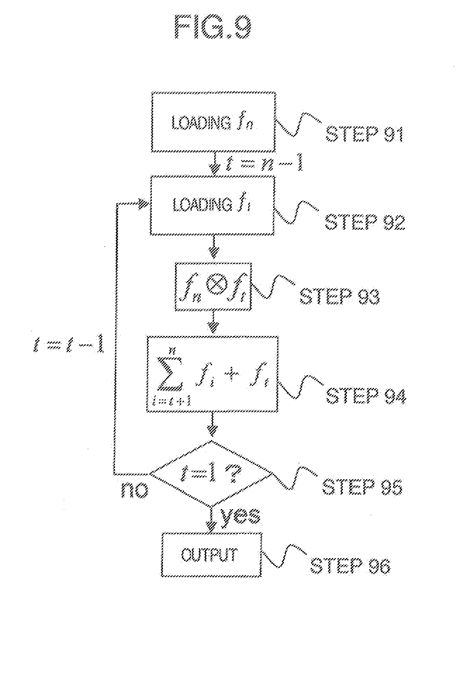

First, referring to a flowchart illustrated in Fig. 9, the explanation will be given below concerning the measurement of the velocity vectors and the methodology for executing the addition processing. Here, it is assumed that the addition number-of-pieces are n pieces, and that the n pieces of image data from $f_1$ to $f_n$ are stored into the frame memory 11 in the order in which they are loaded therein. First of all, the newest image $f_n$ fetched into the ultrasonic diagnostic apparatus is loaded as a reference image for the addition processing (step 91). Subsequently, the 1-frame-previous image $f_t$ (i.e., the initial value of t is equal to (n-1)), which becomes the addition target, is loaded from the frame memory 11 (step 92). Then, using these two pieces of images, the measurement of a velocity vector which has occurred therebetween is performed (step 93). Moreover, based on this measurement result, the movement of the object which has occurred between these two pieces of images is corrected, then performing the addition processing (step 94). The measurement of the velocity vector is performed using the generally-known pattern-matching methodologies, such as mutual correlation method and least-squares method, and the methodologies for the measurement are not particularly limited. Furthermore, the steps ranging from the step 92 to the step 94 are repeated (n-1) times, i.e., until the value of t has become equal to t = 1 (step 95). Finally, the accumulation image, which is acquired by applying the addition processing to the n pieces of images, is outputted to the blood-flow image extracting unit 13 (step 96).

The blood-flow image extracting unit 13 creates an addition blood-flow image in which only a blood-flow region is extracted out of the accumulation image. In the extraction of the blood-flow region, the blood-flow region is judged based on the brightness of the accumulation image. The brightness from the reflection-echo sources contained in the blood flow are accumulated on the reference image for the addition processing by the execution of the addition processing. On account of this accumulation, a flow-trace line resulting from tracing the movement of a reflection-echo source is described on the addition blood-flow image. Meanwhile, the fine structure of the tissue region is maintained even on the accumulation image. This is because the movement of the tissue region is sufficiently slower as compared with the movement of the blood flow. Accordingly, extracting the low-frequency component out of the accumulation image makes it possible to create the blood-flow image. Also, the methodologies as described in the first embodiment are applicable, such as the methodology of including the low-frequency-component ultrasound into the wave-transmission sequence, and the methodologies of using the high-frequency component ultrasound and a contrast agent. Each of these methodologies is a one of utilizing the difference in the brightness between the tissue region and the blood-flow region. Consequently, the application of the addition processing makes it possible to magnify the difference in the brightness even further.

Moreover, the addition blood-flow image is transmitted to the histogram unit 14. The unit 14 calculates the frequency distribution table of the brightness of the addition blood-flow image, using basically the same methodology as the one explained in the first embodiment. On the addition blood-flow image, the distribution of the brightness, which has occurred due to the differences in the blood-flow quantity and the blood-flow velocity, is clearer as compared with the distribution of the brightness in the case of the single image. As a result, it becomes much easier to make the judgment on an region equivalent to the stagnation region from the frequency distribution table.

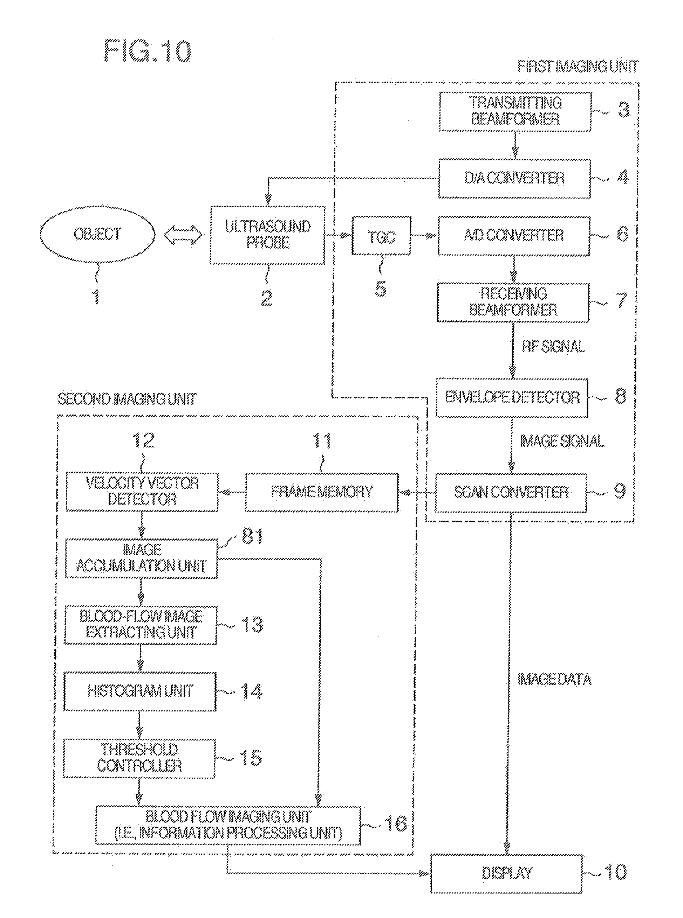

Furthermore, in the threshold controller 15, the operator inputs a threshold value for implementing the color mapping, using the frequency distribution table and the methodology explained in the first embodiment. In addition, in the blood flow imaging unit 16, the color-coded blood-flow stagnation region and the blood-flow velocity vector are superimposed on the image data $f_n$ loaded from the frame memory 11. This processing allows the configuration of the blood-flow distribution image. At this time, the image on which the stagnation region and velocity vector are to be superimposed is also allowed to be the accumulation image created in an image accumulation unit 81, as is illustrated in Fig. 10. The operator can freely select either of the image data $f_n$ and the accumulation image.

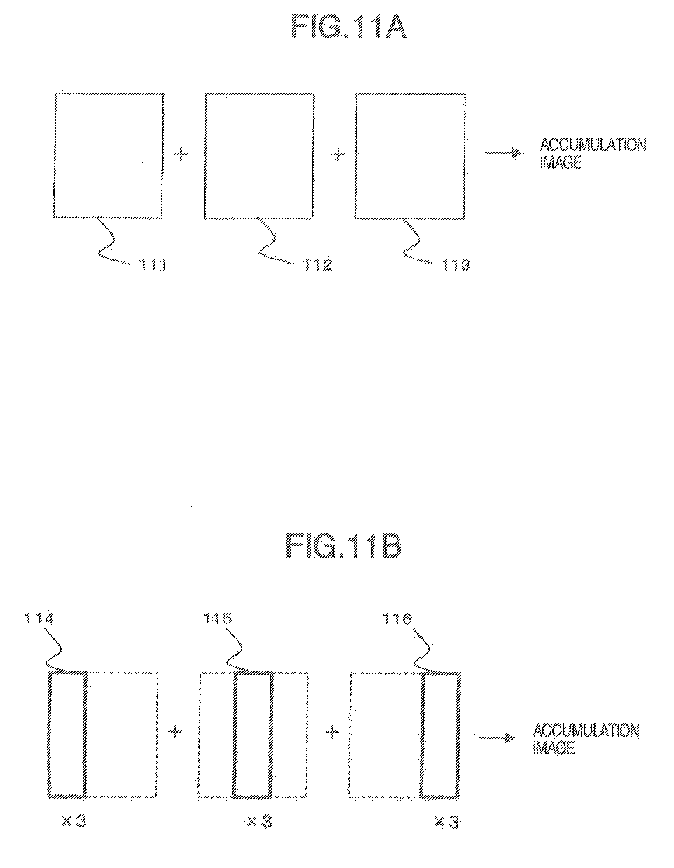

In the case of measuring a velocity vector, if the fetching time for the image data is long, the following possibilities exists: A change in the brightness which occurs between the image data may become larger. Also, a measurement error for the velocity vector may occur. Then, when the accumulation image acquired using the plural pieces of image data is used for the calculation of the frequency distribution table, adjusting the wave-transmission sequence makes it possible to reduce the error in the region searching. Hereinafter, for simplicity, the explanation will be given selecting a case where, as illustrated in Fig. 11A and Fig. 11B, three pieces of images will be added to each other. The numerals 111, 112, and 113 denote image data 1, image data 2, and image data 3 which are acquired using the conventional wave-transmission sequence. In the case of the ordinary 3-frame addition, the measurement of a velocity vector is performed between the respective images, then performing the addition processing based on this measurement result. Meanwhile, in the case of another 3-frame addition, the photographing region is divided (into 3 regions, here) in a direction in which the ultrasound is to be electronically scanned. Then, the transmission/reception of the ultrasound is performed by the amount of the addition number-of-pieces (3 times, here) in each region. This processing makes it possible to reduce the measurement error for the velocity vector without changing the processing time needed for the configuration of the accumulation image. First, the 3-time transmission/reception of the ultrasound is performed in the division region 1 (114), then performing the measurement of the velocity vector and the addition processing based on this measurement result. Subsequently, in the other division region 2 (115) and division region 3 (116) as well, the accumulation image for each division region is created similarly. Finally, the entire accumulation image is created. In comparison with the conventional methodologies, the transmission/reception of the ultrasound is performed in the narrower regions. Accordingly, in each division region, a time difference which occurs between the images is short, and a position shift to be corrected is small. As a result, the measurement accuracies of the velocity vectors are enhanced. Also, the regions to be searched for can be made smaller, which makes it possible to shorten the processing time needed for searching for the matching regions. If an region in which the blood-flow distribution is to be measured is narrow, or if deformation of the object is small, limiting the measurement of the velocity vector into a certain specific region allows implementation of the shortening in the processing time. The velocity vector is measured in at least a single segmentalized region of the tissue region, and the accumulation image including all the image regions is configured based on this measurement result. As described earlier, the extraction of the blood-flow region is made possible by extracting the low-frequency component out of the accumulation image. Consequently, the implementation of a tremendous time shortening is made possible in comparison with the case where the measurement of the velocity vector is performed in all the image regions.

In the case of a normal blood vessel, the streamline of a blood flow substantially becomes a straight line. Meanwhile in the case where there exists an obstruction object such as a plaque, the streamline becomes a curved line or a vortex-like line. This streamline not only indicates the presence or absence of a plaque, but also is useful for identifying the location of "stagnation" at which a blood flow stagnates. The stagnation of a blood flow possesses a possibility of becoming the cause for formation of a blood clot, or a possibility of enhancing the growth rate of the plaque. Accordingly, imaging the stagnation allows implementation of an important diagnosis tool oriented for a preventive diagnosis against the plaque.

According to the above-described respective embodiments, in order to image a stagnation region, it becomes possible to measure the velocity vector of a blood flow, to visualize a vortex-like or curved streamline, and to measure the location at which the blood flow stagnates.

In the CFM (: color flow mapping) method, i.e., the well-known method for measuring the velocity distribution of a blood flow, the direction and size of a blood flow are displayed in a manner of being color-coded on the image. As a result, the CFM method makes it easy to distinguish the stagnation of a blood flow. Nevertheless, the problem of its insufficient spatial resolution remains unsolved, considering a point that the measurement direction is limited, and a point that the average velocity within a constant region is measured. Namely, the spatial resolution provided by the CFM method is insufficient in order to distinguish a stagnation region which occurs at the rear portion of a plaque or at meander portion of a blood vessel. On the B-mode image photographed using a high-frequency ultrasound probe, it is possible to distinguish a stagnation region on the basis of the movement of the reflectors within the blood flow. It is difficult, however, to quantitatively distinguish the stagnation situation of the blood flow. Namely, in many cases, quantitatively distinguishing the stagnation situation varies, depending on a subjective judgment by the observer. Also, in a situation where an intermediate-or-long-term observation is necessary, the variation in diagnosis by the operator and a lowering in the reproducibility can become a serious problem in determining a timing for taking a preventive measure before the stagnation region results in the occurrence of myocardial infarction and cerebral infarction. According to the above-described respective embodiments, it becomes possible to obtain the image display which permits the observer to judge the concentration and stagnation location of a blood flow easily and objectively.

In trying to judge the stagnation region of a blood flow, the brightness distribution from the ultrasound reflection-echo sources, such as hemoglobin and lipid contained in the blood flow, becomes useful information. The low-velocity region of a blood flow or the stagnation region thereof becomes a high-brightness region on the image data. Accordingly, widening a time width needed for the photographing allows implementation of further clarification between the high-brightness region and the low-brightness region.

The above-described description has been given in accompaniment with the embodiments. It is apparent for those who are skilled in the art, however, that the present invention is not limited thereto, and that a variety of modifications and amendments can be made within the spirit of the present invention and the scope of the appended claims. EMBODIMENT 3

The blood-flow image (which, hereinafter, will be referred to as "CFM (: color flow mapping) image") using the general-purpose-intended autocorrelation operation is applied and integrated into the device configuration and processing steps described in the first embodiment. This CFM-image-integrated configuration allows implementation of an image configuration on which the difference in the blood-flow mode is reflected more effectively.

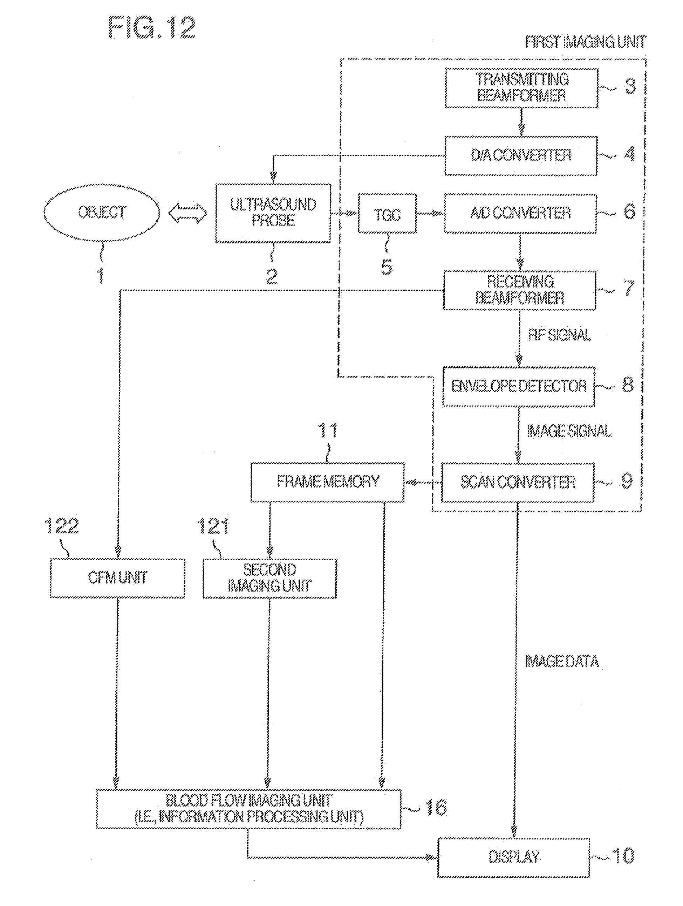

Fig. 12 is a block diagram for illustrating a device configuration example of an ultrasonic diagnostic apparatus according to a third embodiment of the present invention. The device configuration illustrated in Fig. 12 is as follows: Based on the block diagram of the device configuration example in the first embodiment illustrated in Fig. 1, the configuration ranging from the velocity vector detector 12 to the threshold controller 15 is simplified as a second imaging unit 121, and a CFM unit 122 is newly provided. The CFM image is configured as follows: The transmission/reception of the ultrasound is performed in plural times with respect to one and the same scanning line. Next, from a pulse signal acquired, the blood-flow-originated Doppler transition frequency is measured. Moreover, the blood-flow average velocity and reflection intensity measured based on the autocorrelation operation are subjected to color-phase modulation or brightness modulation, thereby configuring the CFM image. Accordingly, the CFM unit 122 illustrated in Fig. 12 basically includes such devices as a mixing unit for measuring the Doppler transition frequency, a MTI (: Moving Target Indicator) for eliminating signals other than the blood flow, and an autocorrelation operation unit for measuring the average velocity and reflection intensity.

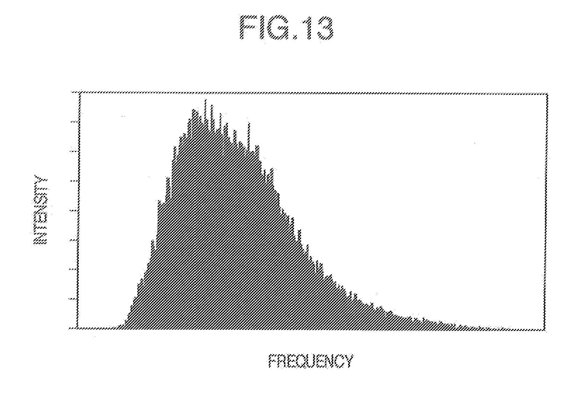

Furthermore, based on the RF signal inputted from the receiving beamformer 7, the CFM unit 122 outputs the Doppler spectrum of the blood flow, i.e., the result of the autocorrelation operation. The area of the Doppler spectrum (Fig. 13), where the vertical axis and the horizontal axis indicate the intensity and the frequency respectively, is correlated with the number of erythrocytes contained in the ultrasound irradiation region. Consequently, the area based on the spectrum is measured for each region provided on each scanning line, and light-and-shade of the color in accordance with the value of the area is displayed on the screen. This processing allows implementation of the imaging of the blood-flow concentration. The CFM image on which the reflection intensity is reflected possesses none of the information in the blood-flow direction. On account of this situation, the blood flow imaging unit (i.e., information processing unit) 16 combines with each other the CFM image, the blood-flow vector measured in the second imaging unit 121, and the tissue image from the frame memory, thereby providing the image for indicating the blood-flow concentration and the blood-flow direction.

BRIEF DESCRIPTION OF THE DRAWINGS

[Fig. 1] Fig. 1 is the block diagram for illustrating the configuration example of the ultrasonic diagnostic apparatus according to the first embodiment of the present invention.
[Fig. 2] Fig. 2 is the flowchart ranging from the measurement of the velocity vector to the configuration of the blood-flow distribution image in the device configuration example described in the first embodiment.
[Fig. 3A] Fig. 3A is the diagram for explaining the measurement methodology for the velocity vector in the device configuration example described in the first embodiment.
[Fig. 3B] Fig. 3B is the diagram for explaining the measurement methodology for the velocity vector in the device configuration example described in the first embodiment.
[Fig. 4A] Fig. 4A is the diagram for explaining the one example of the methodologies for optimizing the searching region in the device configuration example described in the first embodiment.
[Fig. 4B] Fig. 4B is the diagram for explaining the one example of the methodologies for optimizing the searching region in the device configuration example described in the first embodiment.
[Fig. 5] Fig. 5 is the diagram for indicating the one example of the measurement result of the velocity vector in the device configuration example described in the first embodiment.
[Fig. 6] Fig. 6 is the diagram for indicating the one example of the frequency distribution table of the brightness in the device configuration example described in the first embodiment.
[Fig. 7] Fig. 7 is the diagram for indicating the one example of the display modes of the blood-flow distribution image in the device configuration example described in the first embodiment.
[Fig. 8] Fig. 8 is the block diagram for illustrating the configuration example of the ultrasonic diagnostic apparatus according to the second embodiment of the present invention.
[Fig. 9] Fig. 9 is the diagram for explaining the measurement methodology for the velocity vector in the device configuration example described in the second embodiment.
[Fig. 10] Fig. 10 is the block diagram for illustrating the second configuration example of the ultrasonic diagnostic apparatus according to the second embodiment of the present invention.
[Fig. 11A] Fig. 11A is the example of the wave-transmission sequence for reducing the measurement error for the velocity vector in the device configuration example described in the second embodiment.

[Fig. 11B] Fig. 11B is the example of the wave-transmission sequence for reducing the measurement error for the velocity vector in the device configuration example described in the second embodiment.

[Fig. 12] Fig. 12 is the block diagram for illustrating the configuration example of the ultrasonic diagnostic apparatus according to the third embodiment of the present invention.

[Fig. 13] Fig. 13 is the example of the Doppler spectrum.

[Fig. 14] Fig. 14 is the block diagram for illustrating the device configuration example including the velocity vector field detector.

[Fig. 15] Fig. 15 is the example of the vector-distribution diagram.

DESCRIPTION OF REFERENCE NUMERALS

| | |
|---|---|
| 1 | object |
| 2 | ultrasound probe |
| 3 | transmitting beamformer |
| 4 | D/A converter |
| 5 | TGC |
| 6 | A/D converter |
| 7 | receiving beamformer |
| 8 | envelope detector |
| 9 | scan converter |
| 10 | display |
| 11 | frame memory |
| 12 | velocity vector detector |
| 13 | blood-flow image extracting unit |
| 14 | histogram unit |
| 15 | threshold controller |
| 16 | blood flow imaging unit |
| 31 | image data $f_1$ |
| 32 | blood vessel on image data $f_1$ |
| 33 | image data $f_2$ |
| 34 | blood vessel on image data $f_2$ |
| 35 | searching region |
| 36 | segmentalized region $subf_2$ |
| 40 | before-optimization segmentalized region |
| 41 | after-optimization segmentalized region |
| 71 | stagnation region |
| 72 | image accumulation unit |
| 111 | image data 1 |
| 112 | image data 2 |
| 113 | image data 3 |
| 114 | division region 1 |
| 115 | division region 2 |
| 116 | division region 3 |
| 121 | second imaging unit |
| 122 | CFM unit |
| 141 | velocity vector field detector |
| 142 | Extracting-region input unit |
| 151 | vector distribution |

**Claims**

1. An ultrasonic diagnostic apparatus, comprising:

an ultrasound probe for transmitting/receiving ultrasound signals to/from an object;
a first imaging unit for configuring plural pieces of first-image data based on said reception signals received by said ultrasound probe;

a velocity vector detector for measuring blood and tissue velocity vectors based on said plural pieces of first-image data inputted from said first imaging unit;

a blood-flow image extracting unit for configuring a blood-flow image based on said velocity vectors measured by said velocity vector detector;

a histogram unit for calculating number of pixels on each brightness basis with respect to said blood-flow image configured by said blood-flow image extracting unit;

a threshold controller for inputting a brightness threshold value;

an information processing unit for displaying pixels and other pixels in a mutually different display mode, and configuring second-image data by adding said pixels to at least said one piece of first-image data, brightness of said pixels being higher than said brightness threshold value; and

a display for displaying said second-image data configured by said information processing unit.

2. The ultrasonic diagnostic apparatus according to Claim 1, wherein
said blood-flow image extracting unit selects a tissue region based on said velocity vectors, and configures said blood-flow image by eliminating tissue-region data from said first-image data.

3. The ultrasonic diagnostic apparatus according to Claim 1, wherein
said information processing unit color-codes said pixels in accordance with said brightness of said pixels, said brightness of said pixels being higher than said brightness threshold value.

4. The ultrasonic diagnostic apparatus according to Claim 1, wherein
said display displays said second-image data in such a manner that said blood-flow velocity vector is superimposed on said second-image data, said blood-flow velocity vector being measured by said velocity vector detector.

5. The ultrasonic diagnostic apparatus according to Claim 1, wherein
said display further displays a frequency distribution table for indicating said number of said pixels on each brightness basis.

6. The ultrasonic diagnostic apparatus according to Claim 1, wherein
said threshold controller sets, as said brightness threshold value, a brightness of an arbitrary region on said first image.

7. The ultrasonic diagnostic apparatus according to Claim 5, wherein
said frequency distribution table and said brightness threshold value are updated in response to acquisition of said first-image data.

8. The ultrasonic diagnostic apparatus according to Claim 1, wherein
said blood-flow image extracting unit memorizes a predetermined velocity-vector threshold value, and defines, as said blood-flow image, a region in which there exists a velocity vector whose value is larger than said predetermined velocity-vector threshold value.

9. The ultrasonic diagnostic apparatus according to Claim 1, wherein
said blood-flow image extracting unit configures said blood-flow image by eliminating a region from said first-image data, said velocity vectors being substantially equal to zero in said region.

10. The ultrasonic diagnostic apparatus according to Claim 1, wherein
said blood-flow image extracting unit selects a tissue region based on said reception signals, said reception signals being acquired by said ultrasound probe in accordance with wave-transmission sequence including irradiation with a low-frequency component.

11. The ultrasonic diagnostic apparatus according to Claim 1, wherein
said velocity vector detector measures a plurality of velocity vectors thereby to calculate an average velocity vector, said blood-flow image extracting unit extracting said blood-flow image based on said average velocity vector.

12. The ultrasonic diagnostic apparatus according to Claim 1, wherein
said velocity vector detector measures a plurality of velocity vectors then to add said plurality of velocity vectors to each other on each region basis.

13. The ultrasonic diagnostic apparatus according to Claim 1, wherein
said threshold controller sets, as said brightness threshold value, a brightness at center of a blood vessel or in proximity to said center in said first-image data.

14. An ultrasonic diagnostic apparatus, comprising:

an ultrasound probe for transmitting/receiving ultrasound signals to/from an object;
a first imaging unit for configuring plural pieces of first-image data based on said reception signals received by said ultrasound probe;
a memory for memorizing at least two pieces of said plural pieces of first-image data, each of said plural pieces of first-image data corresponding to each of said plural reception signals ranging from said 1st signal to said n-th signal and received by said ultrasound probe;
a velocity vector detector for measuring blood and tissue velocity vectors from said first-image data corresponding to said (n-1)-th signal and said first-image data corresponding to said n-th signal, these two pieces of first-image data being inputted from said first imaging unit and being memorized into said memory;
an image accumulation unit for creating an accumulation image by adding said plural pieces of first-image data to each other;
a blood-flow image extracting unit for extracting a blood-flow image based on said velocity vectors measured by said velocity vector detector, or based on said accumulation image created by said image accumulation unit;
a histogram unit for calculating number of pixels on each brightness basis with respect to said accumulation image;
a threshold controller for inputting a brightness threshold value;
an information processing unit for color-mapping pixels whose brightness are higher than said brightness threshold value, and configuring second-image data by adding said pixels to at least said one piece of first-image data; and
a display for displaying said second-image data configured by said information processing unit.

15. The ultrasonic diagnostic apparatus according to Claim 14, wherein
said image accumulation unit creates said accumulation image by correcting movement of said object based on said tissue velocity vector.

16. The ultrasonic diagnostic apparatus according to Claim 14, wherein
said memory memorizes said plural pieces of first-image data, each of said plural pieces of first-image data corresponding to each of said plural reception signals ranging from said 1st signal to said n-th signal and received by said ultrasound probe,
said image accumulation unit creating said accumulation image by adding to each other said plural pieces of first-image data ranging from said 1st first-image data to said n-th first-image data.

17. The ultrasonic diagnostic apparatus according to Claim 14, wherein
said velocity vector detector acquires said plural pieces of first-image data on a one-piece-by-one-piece basis then to measure said blood and tissue velocity vectors.

18. The ultrasonic diagnostic apparatus according to Claim 14, wherein
said ultrasound probe performs said ultrasound transmission/reception at plural times for each division region basis, said division region being acquired by dividing said plural pieces of first-image data, said plural times being equal to number-of-pieces of said plural pieces of first-image data added by said image accumulation unit.

19. The ultrasonic diagnostic apparatus according to Claim 14, wherein
said blood-flow image extracting unit extracts said blood-flow image by extracting a low-frequency component from said accumulation image.

20. The ultrasonic diagnostic apparatus according to Claim 14, wherein
said blood-flow image extracting unit extracts said blood-flow image by extracting a high-brightness region from said accumulation image.

21. The ultrasonic diagnostic apparatus according to Claim 14, wherein
said velocity vector detector calculates an addition velocity vector by measuring a plurality of velocity vectors and adding said velocity vectors to each other on each region basis,

said blood-flow image extracting unit extracting said blood-flow image in such a manner that a velocity difference between a tissue region and a blood-flow region is emphasized using said addition velocity vector.

**22.** The ultrasonic diagnostic apparatus according to Claim 14, wherein
said blood-flow image extracting unit extracts an addition blood-flow image based on said velocity vectors, said addition blood-flow image being acquired by extracting only a blood-flow region from said accumulation image.

# FIG.1

FIRST IMAGING UNIT

TRANSMITTING BEAMFORMER ~ 3

D/A CONVERTER ~ 4

OBJECT ⟷ ULTRASOUND PROBE → TGC → A/D CONVERTER ~ 6

1    2    5

RECEIVING BEAMFORMER ~ 7

RF SIGNAL

ENVELOPE DETECTOR ~ 8

IMAGE SIGNAL

SECOND IMAGING UNIT

12    11

VELOCITY VECTOR DETECTOR ← FRAME MEMORY ← SCAN CONVERTER ~ 9

BLOOD-FLOW IMAGE EXTRACTING UNIT ~ 13

HISTOGRAM UNIT ~ 14

IMAGE DATA

THRESHOLD CONTROLLER ~ 15

BLOOD FLOW IMAGING UNIT (I.E., INFORMATION PROCESSING UNIT) ~ 16

DISPLAY ~ 10

# FIG.2

```
┌─────────────────────────────────┐
│   VELOCITY VECTOR DETECTION      │── STEP 21
│  BY SUM OF ABSOLUTE DIFFERENCE   │
└─────────────────────────────────┘
              │
              ▼
┌─────────────────────────────────┐
│    DETECTING BLOOD FLOW IMAGE    │── STEP 22
└─────────────────────────────────┘
              │
              ▼
┌─────────────────────────────────┐
│        MAKING HISTOGRAM          │── STEP 23
└─────────────────────────────────┘
              │
              ▼
┌─────────────────────────────────┐
│      SETTING THRESHOLD TO        │── STEP 24
│  DETECT THE PIXELS TO BE COLOR   │
└─────────────────────────────────┘
              │
              ▼
┌─────────────────────────────────┐
│     COLOR MAPPING ON REGION      │── STEP 25
│    HAVING HIGHER BRIGHTNESS      │
│         THAN THRESHOLD           │
└─────────────────────────────────┘
              │
              ▼
┌─────────────────────────────────┐
│     CONSTRUCTING BLOOD FLOW      │
│  IMAGE IN WHICH VELOCITY VECTOR  │
│ IS SUPERIMPOSED ON IMAGE WHERE   │── STEP 26
│    BLOOD-FLOW REGIONS ARE        │
│    COLOR-MAPPED ON EACH          │
│        BRIGHTNESS BASIS          │
└─────────────────────────────────┘
```

## FIG.3A

## FIG.3B

## FIG.4A

## FIG.4B

## FIG.5

## FIG.6

# FIG.7

## FIG.8

FIRST IMAGING UNIT

TRANSMITTING BEAMFORMER — 3

D/A CONVERTER — 4

OBJECT ⟷ ULTRASOUND PROBE → TGC → A/D CONVERTER — 6

1   2   5

RECEIVING BEAMFORMER — 7

RF SIGNAL

SECOND IMAGING UNIT

ENVELOPE DETECTOR — 8

IMAGE SIGNAL

VELOCITY VECTOR DETECTOR — 12

FRAME MEMORY — 11

SCAN CONVERTER — 9

IMAGE ACCUMULATION UNIT — 81

BLOOD-FLOW IMAGE EXTRACTING UNIT — 13

HISTOGRAM UNIT — 14

IMAGE DATA

THRESHOLD CONTROLLER — 15

BLOOD FLOW IMAGING UNIT (I.E, INFORMATION PROCESSING UNIT) — 16

DISPLAY — 10

## FIG.9

```
┌─────────────────┐
│  LOADING fₙ     │ ～ STEP 91
└─────────────────┘
         │  t = n − 1
         ▼
┌─────────────────┐
│  LOADING fₜ     │ ～ STEP 92
└─────────────────┘
         │
         ▼
┌─────────────────┐
│   fₙ ⊗ fₜ       │ ～ STEP 93
└─────────────────┘
         │
         ▼
┌─────────────────┐
│   n             │
│   Σ  fᵢ + fₜ    │ ～ STEP 94
│  i=t+1          │
└─────────────────┘
         │
         ▼
      ╱───────╲
     ╱ t = 1 ? ╲ ～ STEP 95
      ╲───────╱
    no │    │ yes
       │    ▼
       │ ┌─────────────┐
       │ │  OUTPUT     │ ～ STEP 96
       │ └─────────────┘
  t = t − 1
```

$t = n - 1$

$f_n \otimes f_t$

$$\sum_{i=t+1}^{n} f_i + f_t$$

$t = 1 \, ?$

$t = t - 1$

FIG.10

FIRST IMAGING UNIT

| TRANSMITTING BEAMFORMER | 3 |
| D/A CONVERTER | 4 |

OBJECT ←→ ULTRASOUND PROBE → TGC → A/D CONVERTER ~ 6

RECEIVING BEAMFORMER ~ 7

1    2    5

RF SIGNAL

ENVELOPE DETECTOR ~ 8

IMAGE SIGNAL

SECOND IMAGING UNIT

12    11

VELOCITY VECTOR DETECTOR ← FRAME MEMORY ← SCAN CONVERTER ~ 9

IMAGE ACCUMULATION UNIT ~ 81

BLOOD-FLOW IMAGE EXTRACTING UNIT ~ 13

HISTOGRAM UNIT ~ 14

THRESHOLD CONTROLLER ~ 15

IMAGE DATA

BLOOD FLOW IMAGING UNIT (I.E., INFORMATION PROCESSING UNIT) ~ 16

DISPLAY ~ 10

23

## FIG.11A

111     112     113

ACCUMULATION IMAGE

## FIG.11B

114     115     116

×3     ×3     ×3

ACCUMULATION IMAGE

EP 2 151 194 A1

## FIG.12

FIRST IMAGING UNIT

TRANSMITTING BEAMFORMER ~ 3

D/A CONVERTER ~ 4

OBJECT ⟷ ULTRASOUND PROBE → TGC → A/D CONVERTER ~ 6

RECEIVING BEAMFORMER ~ 7

RF SIGNAL

ENVELOPE DETECTOR ~ 8

IMAGE SIGNAL

11

FRAME MEMORY ← SCAN CONVERTER ~ 9

1

2

5

122

121

CFM UNIT

SECOND IMAGING UNIT

IMAGE DATA

BLOOD FLOW IMAGING UNIT
(I.E., INFORMATION PROCESSING UNIT) ~ 16

DISPLAY ~ 10

25

## FIG.13

## FIG.14

FIRST IMAGING UNIT

TRANSMITTING BEAMFORMER ~ 3

D/A CONVERTER ~ 4

OBJECT ⟷ ULTRASOUND PROBE

TGC ~ 5

A/D CONVERTER ~ 6

1

2

RECEIVING BEAMFORMER ~ 7

RF SIGNAL

SECOND IMAGING UNIT

ENVELOPE DETECTOR ~ 8

IMAGE SIGNAL

12

11

VELOCITY VECTOR DETECTOR

FRAME MEMORY

SCAN CONVERTER ~ 9

BLOOD-FLOW IMAGE EXTRACTING UNIT ~ 13

VELOCITY VECTOR FIELD DETECTOR ~ 141

IMAGE DATA

EXTRACTING-REGION INPUT UNIT ~ 142

BLOOD FLOW IMAGING UNIT (I.E., INFORMATION PROCESSING UNIT) ~ 16

DISPLAY ~ 10

# FIG.15

<table>
<tr><td colspan="2" align="center">**INTERNATIONAL SEARCH REPORT**</td><td>International application No.<br>PCT/JP2008/052048</td></tr>
</table>

A. CLASSIFICATION OF SUBJECT MATTER
*A61B8/06*(2006.01)i

According to International Patent Classification (IPC) or to both national classification and IPC

B. FIELDS SEARCHED

Minimum documentation searched (classification system followed by classification symbols)
A61B8/06

Documentation searched other than minimum documentation to the extent that such documents are included in the fields searched
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | JP 8-33625 A (Hitachi Medical Corp.),<br>06 February, 1996 (06.02.96),<br>Full text; Figs. 7 to 9<br>(Family: none) | 1-10<br>11-22 |
| Y<br>A | JP 11-206770 A (Fujitsu Ltd.),<br>03 August, 1999 (03.08.99),<br>Par. No. [0047]<br>(Family: none) | 1-10<br>11-22 |
| Y<br>A | JP 11-299785 A (Fujitsu Ltd.),<br>02 November, 1999 (02.11.99),<br>Par. No. [0041]<br>(Family: none) | 1-10<br>11-22 |

[X] Further documents are listed in the continuation of Box C.  [ ] See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>22 February, 2008 (22.02.08) | Date of mailing of the international search report<br>04 March, 2008 (04.03.08) |
|---|---|
| Name and mailing address of the ISA/<br>Japanese Patent Office | Authorized officer |
| Facsimile No. | Telephone No. |

Form PCT/ISA/210 (second sheet) (April 2007)

| | **INTERNATIONAL SEARCH REPORT** | International application No. |
|---|---|---|
| | | PCT/JP2008/052048 |

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 62-114539 A (Fujitsu Ltd.),<br>26 May, 1987 (26.05.87),<br>Page 3, upper right column, lines 15 to 19;<br>Fig. 1<br>& US 4790321 A & EP 226044 A2 | 1-10<br>11-22 |
| Y<br>A | JP 1-164355 A (Hewlett-Packard Co.),<br>28 June, 1989 (28.06.89),<br>Page 3, upper left column, lines 1 to 5<br>& US 4850364 A & EP 316200 A2 | 1-10<br>11-22 |
| Y<br>A | JP 6-319737 A (Fujitsu Ltd.),<br>22 November, 1994 (22.11.94),<br>Full text; Fig. 6<br>(Family: none) | 1-10<br>11-22 |
| A | JP 5-305087 A (Toshiba Corp.),<br>19 November, 1993 (19.11.93),<br>Full text; Fig. 2<br>(Family: none) | 1-22 |
| A | JP 6-90955 A (Hitachi Medical Corp.),<br>05 April, 1994 (05.04.94),<br>Full text; all drawings<br>(Family: none) | 1-22 |
| A | JP 2002-224115 A (Hitachi Medical Corp.),<br>13 August, 2002 (13.08.02),<br>Full text; all drawings<br>(Family: none) | 1-22 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007117954 A
- JP 3370780 B